# EUROPEAN PATENT APPLICATION

(11) **EP 4 070 760 A1**
(43) Date of publication of application: **12.10.2022**
(21) Application number: 21305438.0
(22) Date of filing: 06.04.2021
(51) Int. Cl.: A61F 2/07, A61F 2/856, A61F 2/06

(54) **VASCULAR PROSTHESIS**

(71) Applicant: Universite d'Aix Marseille, 13284 Marseille Cedex 07 (FR); Assistance Publique Hôpitaux de Marseille, 13005 Marseille (FR); Universite Gustave Eiffel, 77420 Champs-sur-Marne (FR); Bypass Solutions, 13006 Marseille (FR)
(72) Inventor: ALIMI, Yves Simon, 13012 Marseille (FR); MOURET, Frédéric, 34170 CASTELNAU LE LEZ (FR); GARITEY, Vincent, 13016 Marseille (FR); BAAS, David, 67000 Strasbourg (FR)
(74) Representative: Osha Liang

(57) **Abstract**

A vascular prosthesis (1) configured to be implanted in a vessel, having substantially a T-shape, comprises a proximal tubular part (10) forming the base of the "T" and a distal tubular part (20) forming the head of the "T". The second end portion (220) of the distal tubular part (20) is formed by a flap (222). The flap (222) is opposed to a circumferential indentation (223) in the deployed position. The structural frame (224) of the flap (222) comprises an arched segment (227) connected at its ends to the intermediate portion (230) of the distal tubular part (20). The arched segment (227) is bendable so as to form a joint for the folding/unfolding movement of the flap (222).

## Description

### TECHNICAL FIELD

The present disclosure concerns, but is not limited to, a vascular prosthesis configured to be implanted in a vessel. The present disclosure also concerns an implantation device comprising such a vascular prosthesis.

### TECHNICAL BACKGROUND

The yearly number of surgical operations involving vascular prostheses, such as anastomosis, rises all the more so as the number of elderly people increases. Conventional procedures for implanting vascular prostheses suffer from defaults related with their accuracy, duration, and complexity.

One way to improve the non-invasiveness of vascular surgery is the absence of clamping during implantation, such as described, for example, in patent document WO 2009/065917. The absence of clamping allows mitigating ischemia. However, because of the blood flow, the introduction, placement and seal of a connection must be done accurately, quickly and simply to avoid major blood loss. Patent document WO 2009/065917 describes a complex implantation technique involving inflation of a balloon, with a high risk of damaging the blood vessel's wall during maneuvers.

### GENERAL PRESENTATION

According to a first aspect of the present disclosure, there is provided a vascular prosthesis configured to be implanted in a vessel. The vascular prosthesis has substantially a T-shape and comprises a proximal tubular part forming the base of the "T" and a distal tubular part forming the head of the "T". The proximal tubular part has a first lumen, the distal tubular part has a second lumen, and the first and second lumens are fluidly connected to form a common lumen.

The distal tubular part comprises a first end portion, a second end portion, and an intermediate portion extending axially between the first and second end portions, the intermediate portion being provided with a lateral window from which the proximal tubular part extends, the lateral window being surrounded by a peripheral frame. The first end portion and the intermediate portion are radially expandable. The second end portion is formed by a flap movable between a folded position in which the flap is folded inside the second lumen and an unfolded position in which the flap is axially deployed, the flap being opposed to a circumferential indentation of the second end portion in the unfolded position. The flap has a structural frame comprising an arched segment. The arched segment is connected at its ends to the intermediate portion, on each side of the peripheral frame, and surrounds a portion of the peripheral frame in the unfolded position, the arched segment being bendable so as to form a joint for the folding/unfolding movement of the flap.

The vascular prosthesis has substantially a T-shape, in the sense that the proximal tubular part forms the base or vertical bar of the "T", and the distal tubular part forms the head or horizontal bar of the "T". The common lumen fluidly connects the proximal and distal tubular parts of the vascular prosthesis. In other words, the common lumen extends through the proximal tubular part and through the distal tubular part, and also has substantially a T-shape. The first lumen extends through the proximal tubular part. The second lumen extends through the distal tubular part, from the first end portion through the intermediate portion and through the second end portion. The first lumen opens to the exterior at the proximal end, or free end, of the proximal tubular part. The second lumen opens to the exterior at the distal ends, or free ends, of the distal tubular part.

In what follows, the term "comprise" is synonym of (means the same as) "include" and "contain", is inclusive and open, and does not exclude other non-recited elements. Moreover, in the present disclosure, when referring to a numerical value, the terms "about" and "substantially" are synonyms of (mean the same as) a range comprised between 80% and 120%, preferably between 90% and 110%, of the numerical value.

The vascular prosthesis is configured to be implanted in a vessel, in particular a blood vessel. For example, the vascular prosthesis may be implanted perpendicularly to a transparietal puncture made in a vessel beforehand, and through which the vascular prosthesis is inserted.

The vascular prosthesis may be suitable for a wide variety of therapeutic uses. Such therapeutic uses may include surgical anastomosis, e.g., side-to-end anastomosis of vessels, such as blood vessels. In the present disclosure, by "anastomosis", it is meant a connection or opening between two cavities or passages that are normally diverging or branching, such as between vessels, e.g., blood vessels. The two cavities or passages may for example branch and form part of a same blood vessel. Blood vessels that are deemed to necessitate surgical anastomosis may for example be subjected to stenosis, *i.e.,* an abnormal occlusion of the vessel.

The vascular prosthesis may allow to perform a surgical anastomosis with a simpler implantation technique. In particular, such a technique may be carried out without using clamps, leading to a so-called "clampless" surgical anastomosis.

In the present disclosure, an axial direction is the direction of the central axis of a tubular part, and a radial direction is a direction perpendicular to said central axis. Similarly, an axial plane is a plane containing the central axis and a radial plane is a plane perpendicular to the central axis. The adverbs "axially" and "radially" refer to the axial and radial direction, respectively. The circumferential direction is the direction along the circumference of the tubular part, in a radial plane. The adverb "circumferentially" refers to the circumferential direction.

As previously mentioned, the flap is movable between a folded position in which the flap is folded inside the second lumen and an unfolded position in which the flap is axially deployed. Thus, the second end portion is movable between at least two positions, *i.e.,* a folded or retracted position and an unfolded or deployed position. Further, the distance between the axial projection of the distal end or tip of the flap, on the one hand, and the virtual point of junction between the head and the base of the "T" on the other hand, is longer in the unfolded position than in the folded position. The meaning of "axially deployed" is thus related to this longer distance in the unfolded position. The virtual point of junction of the two bars of the "T" in the prosthesis is considered as the reference point in both positions: when the flap is in the folded position and when the flap is in the unfolded position. The "axial projection of the distal end of the flap" is the orthogonal projection on the central axis of the distal tubular part forming the head of the "T".

Such a configuration of the vascular prosthesis in which the flap is folded inside the second lumen allows minimizing the space occupied by the distal tubular part of the vascular prosthesis prior to its implantation in a vessel. This minimized size of the prosthesis is useful during a surgical intervention, by facilitating the prosthesis implantation and/or by reducing the invasiveness of surgery. For example, the minimized size may facilitate the incorporation of the prosthesis into a device for implanting the prosthesis, for example an introduction catheter, and also facilitate the penetration and positioning of said device within a vessel. For example, such a configuration of the vascular prosthesis may allow to implant the prosthesis in the vessel without having to pull on the prosthesis toward the downstream part of the vessel (in order to ensure blood tightness), thereby mitigating the trauma to the vessel.

The vascular prosthesis thus aims at improving conventional side-to-end anastomosis techniques, by providing, among other advantages, a less invasive surgical approach. Compared to classic surgical anastomosis, the use of such a vascular prosthesis may offer potential advantages including, for example, the avoidance of skin incision or dissection, the avoidance of the use of arterial clamps (upstream and downstream the vessel), the avoidance of a suture by a vascular thread, the avoidance of the placement of a drain, and/or the reduction in the duration of anastomosis. During the prosthesis implantation, when no clamp is used to interrupt blood flow, the blood flux goes from upstream of the vessel to downstream of the vessel. Reduction in the duration of anastomosis decreases ischemia of the organs downstream of the vessel in which the vascular prosthesis is configured to be implanted, while, at the same time, reducing systemic vascular resistance or afterload.

The foregoing positive effects stemming from the design of the vascular prosthesis are accompanied by a substantial decrease in complication rates and shortening of the hospital stays, e.g., divided by two, which represents a further step towards ambulatory surgery.

The size of the vascular prosthesis may be adapted to the vessel's dimensions in which the vascular prosthesis is configured to be implanted. For example, the diameter or width of the distal tubular part may be comprised between 1 mm and 50 mm, in particular between 2 mm and 25 mm.

The first end portion and the intermediate portion are radially expandable, thereby allowing the distal tubular part of the prosthesis to adhere to a vessel wall in which the prosthesis is implanted. For example, the first end portion and the intermediate portion may both comprise a structural frame, e.g., a stent or part of a stent, comprising a self-expandable material. Said self-expandable material may be, for example, a metal alloy, such as a nickel titanium alloy (also known as nitinol), which exhibits a shape memory effect and superelasticity at different temperatures.

The second end portion of the vascular prosthesis is formed by a flap. The "flap" or "lateral flap" is a part of the vascular prosthesis that is hinged or attached on one side of the intermediate portion, is folded or "hangs down" from its attachment in the folded position, and is unfolded or "lifted up" during the movement of the flap from the folded position to the unfolded position. The flap aims, among other goals, at covering a part of a vessel wall in which the prosthesis is implanted, thereby allowing the second end portion of the distal tubular part of the prosthesis to adhere to said vessel wall.

The flap has a structural frame forming the "backbone" of the flap. In particular, when the flap is covered with a covering layer or graft, it is understood that the covering layer does not form part of the structural frame.

The structural frame of the flap comprises an arched segment connected at its ends to the intermediate portion on each side of said peripheral frame. The arched segment surrounds a portion of the peripheral frame in the unfolded position. The arched segment is bendable so as to form a joint, or hinge, for the folding/unfolding movement of the flap. In other words, the folding/unfolding movement of the flap is made possible by bending/unbending the arched segment.

The flap is opposed to a circumferential indentation, or scallop, in the unfolded position. By "opposed", it is meant that, in the unfolded position, the flap is radially opposed to the indentation in a radial cross-section of the second end portion. In the unfolded position, depending on the axial position along the second end portion, the circumferential indentation may be present on, for example, about 50% to more than 99% of the total circumference of the second end portion.

The lateral window of the intermediate portion has a peripheral frame. The peripheral frame forms part of the structural frame or "backbone" of the intermediate portion. The lateral window is surrounded by a peripheral frame. The peripheral frame stiffens the periphery of the lateral window, thereby providing support for the connection between the proximal tubular part and the distal tubular part. The peripheral frame may have a rounded shape, an oval shape, an ellipsoid shape, a rectangular shape or a more irregular shape. In certain embodiments, the peripheral frame is symmetrical or substantially symmetrical with respect to the axial plane of the distal tubular part that contains the center of the lateral window.

In addition to the peripheral frame, the structural frame or "backbone" of the intermediate portion comprises a support frame. The support frame may comprise stent struts. The support frame may or may not be directly connected to the peripheral frame. By "directly connected", it is meant that a structural linkage, e.g., stent struts, is provided between the support frame and the peripheral frame. Thus, the peripheral frame is stabilized and cannot be easily decoupled from the rest of the vascular prosthesis. In certain embodiments, the support frame is directly connected to the peripheral frame, on the opposite sides of the peripheral frame in the circumferential direction. In certain embodiments, the general shape of the support frame is tubular and, in radial cross section, the support frame forms an open annular ring. In certain embodiments, the support frame occupies about the whole circumference of the intermediate portion that is left over by the peripheral frame.

The structural frame of the flap comprises an arched segment connected at its ends to the intermediate portion on each side of said peripheral frame, thereby surrounding a portion of the peripheral frame in the unfolded position. The arched segment is inherently curved and, in the unfolded position, the curvature of the arched segment surrounds or encircles a part of the peripheral frame, i.e., the part of the peripheral frame which is the closest to the arch. The word "surrounds" echoes the design of the arched segment which has, for example, a parabolic shape, a U-shape or a V-shape.

The arched segment is connected on each side of the peripheral frame. The connection is made at the ends of the arched segment, which are circumferentially offset, *i.e.,* are arranged at different positions on the circumference of the second end portion. In particular, the ends of the arched segment may be arranged on opposite sides of the circumference. For example, these ends may be arranged symmetrically with respect to the axial plane of the distal tubular part that contains the central axis of the proximal tubular part. The two circumferentially offset ends form the two ends of the "U", "V", or parabola formed by the arched segment. The apex of the "U", "V", or parabola, i.e., the middle of the arched segment, is the part of the arched segment closest to the tip or extremity of the flap.

It is noteworthy that the structural connection between the arched segment and the intermediate portion is made on each side of the peripheral frame and not on the peripheral frame, i.e., the connection points are arranged on the support frame of the intermediate portion. The arched segment is thus directly connected to the support frame but not directly connected to the peripheral frame. By "directly connected", it is meant that a structural linkage is provided. Accordingly, there is no structural linkage between the arched segment and the peripheral frame. Such a configuration combined with the fact that the arched segment forms a kinematic joint, or hinge, for the folding/unfolding movement of the flap, prevents irreversible plastic deformation, or plastification, of the peripheral frame during the movement of the flap. In other words, the arched segment (or parts of it) takes up the bending stresses during the folding/unfolding of the flap, thereby preserving the peripheral frame and the connection between the proximal and distal parts of the prothesis.

Due to the absence of direct connection between the arched segment and the peripheral frame, there is a gap in the structural frame or backbone of the distal tubular part, in the axial direction. Such a gap is an area of the flap devoid of structural linkage but which may be covered, for example, with graft. This configuration facilitates the folding of the flap and prevents plastification of the peripheral frame such as described above.

The arched segment facilitates the unfolding or deployment of the flap. The arched segment further facilitates the adherence of the flap to the vessel wall in which the vascular prosthesis is to be implanted, by creating a radial force towards the vessel wall.

In certain embodiments, the arched segment is elastically bendable between an unstable bent position corresponding to the folded position of the flap and a stable unbent position corresponding to unfolded position of the flap. The arched segment is thus elastically deformable between an unstable state and a stable state, and the deformation of the arched segment from the unstable state to the stable state makes the flap move from the folded position to the unfolded position. It is noteworthy that the plastification or plastic deformation of the arched segment before the deployment of the vascular prosthesis should be avoided. In other words, the arched segment should be able to undergo a reversible change of shape in response to applied forces during the bending.

In certain embodiments, the vascular prosthesis comprises at least one covering layer or graft, *i.e.,* a fabric having substantially a circular cross-sectional configuration and running along the "T" of the vascular prosthesis, either partly or totally along the surface of the "T". In certain embodiments, the graft may comprise a material which is waterproof and blood tight. Such a material may for example be selected in the group comprising polytetrafluoroethylene (PTFE), a woven or knitted polyester (e.g., Dacron^{®}).

In certain embodiments, the vascular prosthesis comprises at least one stent. In the present disclosure, by "stent", it is meant a tubular frame, for example a frame comprising struts. Such a tubular frame is not necessarily closed circumferentially. The at least one stent may be covered and embedded in the foregoing covering layer or graft. The at least one stent may be attached to the graft, *e.g.,* by adhesive or by suturing.

In certain embodiments, in the unfolded position, the circumferential width of the flap decreases with the distance from the intermediate portion. By "circumferential width", it is meant the distance separating two points at the edge of the flap, on each opposite side of the flap in the circumferential direction. In combination with the arched segment, such a design of the flap improves the adherence of the prosthesis on the vessel wall. In particular, the flap edge may have a parabolic shape. Such a parabolic shape allows increasing the amount of graft nearby the lateral window of the intermediate portion, thereby improving the tightness or impermeability of the prosthesis, for example the blood tightness (i.e., the tightness to blood leakage or circulation between the prosthesis and the vessel wall).

In certain embodiments, the circumferential distance along the flap between the ends of the arched segment is comprised between 10% and 50% of the total circumference of the second lumen. Being above 50% starts hindering the folding of the flap. Conversely, being below 5% increases the risks of plastification of the lateral window during the folding of the flap. For example, said circumferential distance is between 20% and 35% of the total circumference of the second lumen.

In certain embodiments, the two ends of the arched segment connected on each side of the peripheral frame are connected to the support frame of the intermediate portion and, in particular, on the edge of the support frame. In other embodiments, said ends are disposed at more central locations of the support frame, for example at a distance comprised between 1% et 30% of the axial distance separating the edge of the support frame and the axial level of the center of the "T".

In certain embodiments, in the deployed position, the flap is outwardly offset with respect to the axial direction of the distal tubular part. By "outwardly offset", it is meant that a non-zero angle α is formed between, on the one hand, a straight line passing through the tip of the flap and the circumferential middle point between the ends of the arched segment, and, on the other hand, the axial direction, or central axis, of the distal tubular part. Such an angle or offset may for example be between 0.5° and 30° with respect to said axial direction, for example between 5° and 20°. Such an offset may for example allow to compensate for the plastification, if present, of certain parts of the prosthesis, by forcing the flap to adhere to the vessel wall, in the deployed position. Said offset stems from the design of the structural frame of the flap, and may result from a general inclination, from an independent curved region or fold, or from cumulative curved regions or folds of the structural frame of the flap.

In certain embodiments, the structural frame of the flap further comprises at least one finger connected to the arched segment. The at least one finger may extend from the arched segment toward the edge of the flap. Additionally, or alternatively, the at least one finger may extend from the arched segment toward the peripheral frame, without however, being directly connected to the peripheral frame, so as to leave a "gap" in the structural frame or backbone of the distal tubular part, as described above. In certain embodiments, the structural frame of the flap comprises a plurality of said fingers disposed at regular intervals on the arch. For example, the fingers may be symmetrically arranged with respect to the axial median plane of the flap.

In certain embodiments, the at least one finger extends from the middle of the arched segment. The length of such a middle finger may be superior to the length of other (peripheral) fingers, when present. The length of the fingers may decrease all the more so as the fingers are offset from the middle of the arched segment.

A parabolic shape of the flap enables increasing the amount of graft nearby the lateral window of the intermediate portion, thereby improving the tightness or impermeability of the prosthesis. At the same time, in order to improve the adherence of the flap to the vessel wall in the deployed position, a certain amount of structural frame of the flap per surface unit of the flap may be needed. In that regard, a configuration of the flap comprising a middle finger and additional symmetrically arranged peripheral fingers - the length of which decreasing with their offset from the middle of the arched segment - allows to obtain an "adhering" parabolic flap, while minimizing the quantity of structural frame per surface unit of the flap, thereby avoiding or mitigating the trauma undergone by the vessel wall. In addition, such a configuration may limit the risks of blocking the flap in the folded position, and facilitates the crimping of the prosthesis into a catheter.

In certain embodiments, the structural frame of the flap comprises at least one rib extending from the at least one finger. Said rib may extend from, e.g., the middle finger. For example, two ribs may extend symmetrically from the middle finger toward the edge of the flap. In certain embodiments, said ribs extend in a curved manner from the middle finger, and the ends of said ribs are oriented toward the intermediate portion as opposed to toward the tip of the flap.

In certain embodiments, the structural frame of the flap comprises at least one finger extending perpendicularly from the arched segment, for example at least three fingers, e.g., five fingers.

In certain embodiments, the at least one finger comprises at least one hole. For example, in certain embodiments, the at least one finger comprises a hole at its extremity, i.e., the at least one finger has a distal end with a hole. Such holes may allow to attach the structural frame of the flap to a covering layer or graft, e.g., by sewing. In addition, the presence of at least one hole may allow the structural frame of the flap to be connected to the sheath of an implantation device via a link passing through the hole, thereby maintaining the flap in the folded position before implantation.

In certain embodiments, the at least one finger comprises at least one widening. By "widening", it is understood a circumferential extension of the finger along the circumference of the second end portion. In certain embodiments, the at least one finger has a distal end with a widening. The at least one widening may thus be positioned at the extremity of the fingers and may for example have a rounded shape, which improves the adherence of the flap to the vessel wall, by offering a "soft" anchoring point of the finger to the vessel wall, in terms of trauma caused to the vessel wall.

In certain embodiments, the structural frame of the flap comprises a plurality of said fingers which do not all have the same rigidity. In certain embodiments, the middle finger is the fingers with the highest rigidity and may for example show one or more widenings or circumferential extensions on its median part.

In certain embodiments, the at least one finger comprises at least one fold, so that, in the deployed position, the finger extends outwardly from the central axis of the distal tubular part. In other words, a non-zero angle β is formed between, on the one hand, a straight line passing through the distal end of the finger and its connection point to the arched segment, and, on the other hand, the axial direction, or central axis, of the distal tubular part. If a peripheral finger comprises a fold, the projection of said straight line on the axial plane of the distal tubular part that contains the center of the lateral window is taken into account to calculate the angle. The resulting angle or offset from the central axis, may for example be between 0.5° and 30°, for example between 5° and 20°. For example, in certain embodiments, the middle finger may comprise two folds, the first fold being close to the arched segment and the second fold being positioned at about the middle of the median part of said middle finger.

According to a second aspect, the present disclosure further concerns an implantation device comprising the foregoing vascular prosthesis with the flap in the folded position. The device according to the second aspect may comprise a sheath and a link between the sheath and the vascular prosthesis. The link may be, for instance, a rod, a rope or a combination thereof.

Other features and advantages of the invention will become apparent from the following detailed description, taken in conjunction with the accompanying drawings, which illustrate, by way of example, the principles of the invention.

### BRIEF DESCRIPTION OF THE DRAWINGS

In the drawings, like reference signs generally refer to the same or like parts throughout the different views. The drawings are not necessarily to scale, emphasis instead generally being placed upon illustrating the principles of the invention.
FIG. 1 is a perspective view of a diagrammatic representation of an example of a vascular prosthesis, which is configured to be implanted in a stenosed blood vessel.
FIG. 2 is a side view of the vascular prosthesis shown in FIG. 1.
FIG.3 is a top view of a diagrammatic representation of the uncovered distal tubular part of the vascular prosthesis shown in FIGs.1-2, with its second end portion in the deployed position.
FIG. 4 is a side view of the distal tubular part shown in FIG. 3.
FIG. 5 is an enhanced top view of the distal tubular part shown in FIGs. 3-4.
FIG. 6A is a side view of a diagrammatic representation of the uncovered distal tubular part of another example of vascular prosthesis, with its flap in the deployed position.
FIG. 6B is a top perspective view of the distal tubular part shown in FIG. 6A.
FIG. 7A is a side view of a diagrammatic representation of the uncovered distal tubular part of yet another example of vascular prosthesis, with its flap in the deployed position.
FIG. 7B is a top perspective view of the distal tubular part shown in FIG. 7A.

### DETAILED DESCRIPTION OF EXAMPLES

Examples of vascular prostheses will now be described in detail with reference to the accompanying figures. In the following detailed description, numerous specific details are set forth in order to provide a more thorough understanding of the invention. However, it will be apparent to one of ordinary skill in the art that the invention may be practiced without these specific details. In other instances, well-known features have not been described in detail to avoid unnecessarily complicating the description.

The following description provides non-limiting examples of vascular prostheses. The description further provides non-limiting details about devices for implanting the vascular prosthesis, as well as details concerning potential applications of such prosthesis.

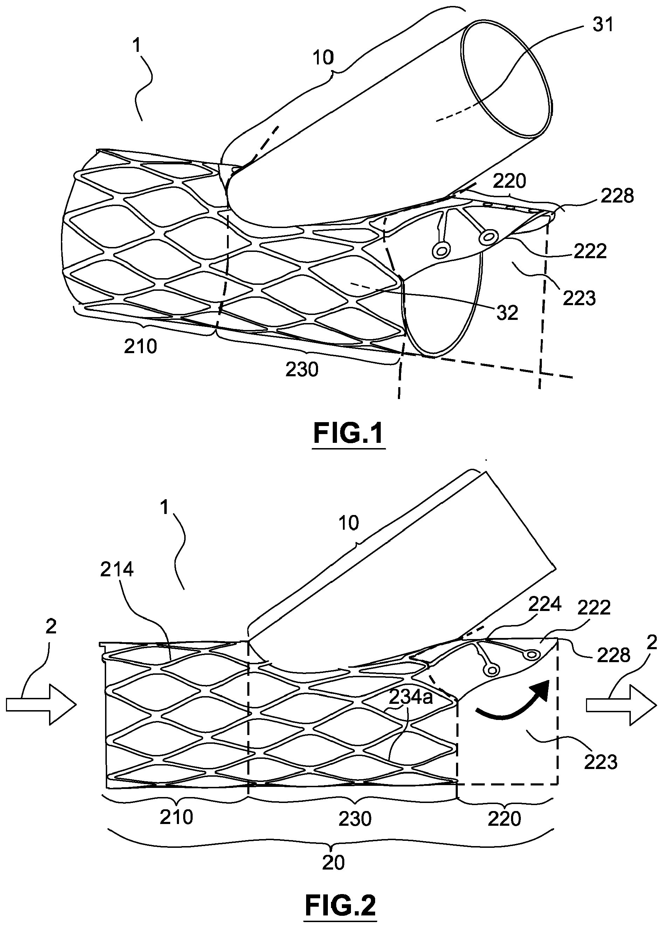

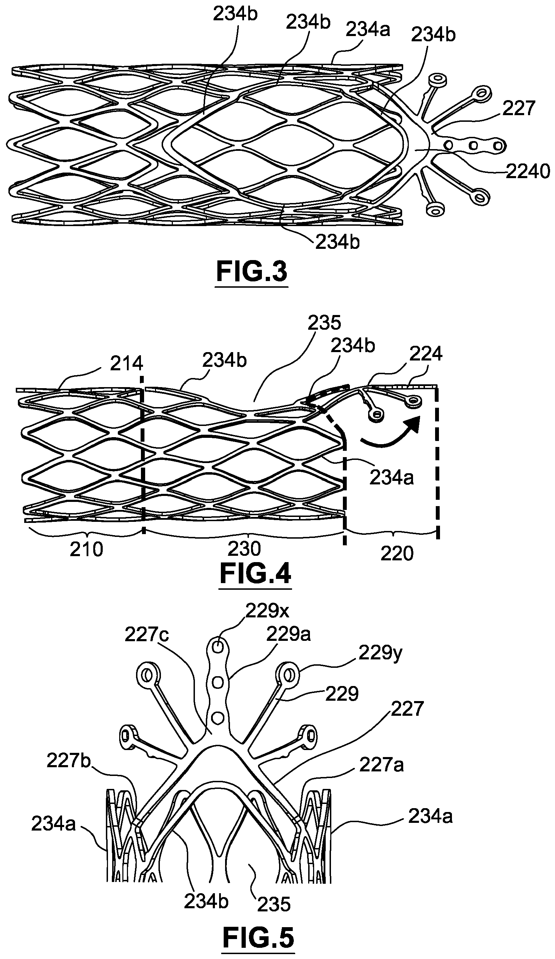

FIG. 1 and FIG. 2 show a perspective view and a side view, respectively, of a vascular prosthesis 1 which is implanted in a stenosed blood vessel (not shown). The (uncovered) distal tubular part 20 of the vascular prosthesis 1 shown in FIGs.1-2 is illustrated in greater detail in FIGs. 3-5.

In the vessel, as shown in FIG. 2, the blood flux 2 goes from the left (upstream) to the right (downstream). The second end portion 220 of the distal tubular part 20 of the vascular prosthesis is positioned downstream, as opposed to the first end portion 210, which is positioned upstream.

As illustrated in FIGs. 1-2, the vascular prosthesis 1 has substantially a T-shape, and comprises a proximal tubular part 10 forming the base of the "T" and a distal tubular part 20 forming the head of the "T". The proximal tubular part 10 has a first lumen 31, the distal tubular part 20 has a second lumen 32, and the first and second lumens 31, 32 are fluidly connected to form a common lumen. The common lumen has substantially a T-shape, the first lumen 31 forming the base of the "T", and the second lumen 32 forming the head of the "T". However, as illustrated, the base of the "T" is not strictly perpendicular to the head of the "T". In other words, the angle formed between the base and the head of the "T" is not strictly equal to 90° and may be comprised between 10° and 90°.

As illustrated in FIGs. 1-2, the distal tubular part 20 comprises a first end portion 210, a second end portion 220, and an intermediate portion 230 extending axially between the first and second end portions. As shown in FIGs. 3-4, the intermediate portion 230 is provided with a lateral window 235 from which the proximal tubular part 10 extends, and said lateral window is surrounded on all its side by a peripheral frame 234b.

The proximal tubular part 10 is connected to the intermediate portion 230. This may be achieved, for example, by a covering layer or graft comprised in the prosthesis and covering both the proximal tubular part 10 and the distal tubular part 20, such as illustrated in FIGs. 1-2. The graft may comprise materials suitable for being included into vascular prostheses configured to be implanted in blood vessels, such as, for example, PTFE.

The first end portion 210 and the intermediate portion 230 of the distal tubular part 20 are radially expandable. This means that the first end portion 210 and the intermediate portion 230 may each move from a radially compressed state to a radially deployed state. In the radially deployed state, the circumference of the first end portion 210 and the intermediate portion 230 is bigger than in the radially compressed state. For example, the first end portion 210 and the intermediate portion 230 may each comprise a tubular frame or stent made of a self-expendable material, such as nitinol.

The intermediate portion 230 is provided with a support frame 234a. The support frame 234a is a tubular frame or stent, which is not closed circumferentially, in order to accommodate the space for the lateral window 235. In this particular example of prosthesis 1, the support frame 234a of the intermediate portion 230 has the shape of an open annular ring in a radial cross-section (not shown) of the intermediate portion 230. In this example, the support frame 234a occupies the whole circumference of the intermediate portion that is left over by the lateral window 235 and the peripheral frame 234b, along the axial length of the intermediate portion. By the "whole circumference", it is of course understood, since the support frame 234a comprises stent struts in this instance, the circumference occupied both by the struts and by the meshes, i.e., the empty spaces formed by the struts.

Moreover, the first end portion 210 is also provided with a tubular frame or stent 214, which in this example is closed circumferentially.

As further shown in FIGs. 1-2, the second end portion 220 is formed by a flap 222 having a structural frame 224, the flap 222 being opposed to a circumferential indentation 223 of the second end portion 220 in the deployed position. The structural frame 224 of the flap may for example be made of the same material as the frame 214 and the support frame 234a. As further shown in FIGs. 1-2, all the different frames 214, 224 and 234a of the distal tubular part 20 may be covered and embedded in a covering layer or graft comprised in the vascular prosthesis.

The flap 222, and therefore the second end portion 220, is movable between a folded (or retracted) position in which the flap is folded inside the second lumen 32 and an unfolded (or deployed) position in which the flap is axially deployed. The prosthesis illustrated in in FIGs. 1-2 both has a deployed position and a retracted position, and is illustrated in the deployed position in the Figures. When the prosthesis goes from its retracted position to its deployed position, the flap 222 may move from its retracted position to its deployed position, as illustrated by the black arrow on FIGS. 2 and 4. Either simultaneously or consecutively, the first end portion 210 and the intermediate portion 230 may radially expand, by moving from their radially compressed state to their radially deployed state.

FIG.3 and FIG. 4 show a top view and a side view, respectively, of the (uncovered) distal tubular part 20 of the vascular prosthesis shown in FIGs. 1-2, with its second end portion 220 in the deployed position. FIG. 5 is an enhanced top view of the distal tubular part shown in FIG. 3. As mentioned above, the distal tubular part 20 also comprises a covering layer or graft which is not shown in FIGs. 3-5.

As shown in FIGs. 3-5, the lateral window 235 of the intermediate portion 230 is surrounded by a peripheral frame 234b. In this example, the peripheral frame 234b follows the perimeter of the window 235 in a continuous way on all sides of the window 235 and has an oval shape. As further shown in the Figures, in this example, the peripheral frame 234b is symmetrical with respect to the axial plane of the distal tubular part 20 that contains the center of the lateral window 235, and its circumferential width decreases with the distance from said center of the lateral window 235. In that manner, more circumferential surface of the intermediate portion 230 is available for the support frame 234a at the proximity of both end portions 210 and 220.

As further shown in FIGs. 3-5, and in particular in the enhanced top view of FIG. 5, the structural frame 224 of the flap 222 comprises an arched segment 227 connected at its ends to the intermediate portion 230 on each side of said peripheral frame 234b. The arched segment 227 thereby surrounds a portion of the peripheral frame 234b. The arched segment 227 is bendable so as to form a joint for the folding/unfolding movement of the flap. The unfolding movement of the flap 222 is illustrated by the black arrow on FIGS. 2 and 4.

In this example, the support frame 234a of the intermediate portion 230 is directly connected to the peripheral frame 234b, on each opposite side of the intermediate portion 230 with respect to the axial plane of the distal tubular part that contains the center of the lateral window 235.

As shown in FIGs. 3 and 5, in this example, the arched segment 227 has a parabolic shape, in the sense that the projection of the arched segment on the axial plane of the distal tubular part 20 that is orthogonal to the central axis of the proximal part 10 - when the two bars of the "T" are orthogonal -, has the shape of a parabola.

The arched segment 227 is connected on each side of the peripheral frame 234b. The connection is made at the ends 227a, 227b of the arched segment 227. Both ends 227a, 227b are circumferentially offset, *i.e.,* are arranged at different positions on the circumference of the second end portion 220. In this example, the ends 227a, 227b are arranged symmetrically with respect to the axial plane of the distal tubular part 20 that contains the center of the lateral window 235. The two circumferentially offset connection points 227a, 227b are the two ends of the parabola of the arched segments 227.

As shown in FIGs. 3-5, the connection of the arched segment 227 on each side of the peripheral frame is not directly made on the peripheral frame 234b, i.e., there is no direct or structural junction of the arched segment 227 to the peripheral frame 234b. Instead, the arched segment 227 is directly connected to the support frame 234a of the intermediate portion 230. In other words, the two ends 227a, 227b of the arched segment 227 are arranged on the support frame 234a. Such a configuration, combined with the fact that the arched segment 227 forms a kinematic joint, or hinge, for the folding/unfolding movement of the flap 222 prevents irreversible plastic deformation - or plastification - of the peripheral frame 234b, during the movement of the flap 222. In other words, the arched segment 227 (or parts of it) takes up the bending stresses during the folding/unfolding of the flap 222, thereby preserving the peripheral frame 234b and the connection between the proximal and distal parts of the prothesis. As shown in the Figures, in this example, the two ends 227a, 227b of the arched segment 227 are arranged on the edge of the support frame 234a with the second end portion 220.

Hence, as shown in FIG. 3, a "gap" 2240 in the structural frame or backbone is present along the axial direction of the distal tubular part 20, between the arched segment 227 and the peripheral frame 234 b, which facilitates the folding of the flap 222, and prevents plastification of the peripheral frame 234b such as described above.

In this example, the arched segment 227 is elastically bendable between an unstable bent position corresponding to the folded position of the flap 222 and a stable unbent position corresponding to unfolded position of the flap 222. The deformation of the arched segment 227 from an unstable state to a stable state makes the flap 222 move from the folded position to the unfolded position. In this manner, plastification of the arched segment 227 before the deployment of the vascular prosthesis can be avoided, and the arched segment 227 is able to undergo a reversible change of shape during the bending.

In this example, in the deployed position, the circumferential width of the flap 222 decreases with the distance from the intermediate portion 230. As shown in FIGs. 1-2, the flap edge has a parabolic shape. Such a parabolic shape allows, for example, to increase the amount of graft nearby the lateral window 235 of the intermediate portion 230, thereby improving the adherence of the prosthesis on the vessel wall, as well as the blood tightness of the prosthesis.

In this example, the circumferential distance along the flap 222 between the ends 227a, 227b of the arched segment 227 is comprised between 20% and 35% of the total circumference of the second lumen 32. Being above 50% would hinder the folding of the flap 222. Conversely, being below 5% would increase the risk of plastification of the peripheral frame 234b of the lateral window 235 during, for example, the folding of the flap 222.

As shown in FIG. 5, in this example, the structural frame 224 of the flap 222 further comprises a plurality of fingers 229 connected to the arched segment 227 and extending perpendicularly from the arched segment 227 toward the edge of the flap 222. In this example, five fingers 229 are disposed at regular intervals on the arched segment 227, and are symmetrically arranged with respect to the axial plane of the distal tubular part 20 that contains the center of the lateral window 235. Moreover, in this example, a middle finger 229a of superior length extends from the middle 227c of the arched segment. The inventors have shown that such a configuration of the structural frame 224 of the flap allows to ensure a good adherence of the flap to the vessel wall in which the prosthesis is configured to be implanted, while minimizing the amount of structural frame of the flap per surface unit of the flap, thereby avoiding or mitigating the trauma undergone by the vessel wall.

In this example, as shown in e.g., FIGs. 3-5, the fingers 229 have distal ends with holes 229x. Such holes 229x may allow to attach the structural frame 224 of the flap to a covering layer or graft, e.g., by sewing. In this example, the middle finger 229a comprises three holes 229x disposed at regular intervals on its length, which allow to link the structural frame 224 of the flap to the sheath of an implantation device according to the second aspect of the present disclosure, thereby maintaining the flap in the folded or retracted position before implantation.

Additionally, in this example, the fingers 229 comprise widenings 229y or circumferential extensions of the fingers positioned at the distal ends of the fingers. In this example, the widenings 229y have a rounded shape, which improves the adherence of the flap 222 to the vessel wall, by offering a "soft" anchoring point of the finger 229 to the vessel wall, in terms of trauma caused to the vessel wall.

Further, in this example, the five fingers 229 do not all have the same rigidity. In this example, the fingers are made of the same material and the difference in rigidity stems from the differences in circumferential width. The middle finger 229a shows two widenings on its median part contrary to the peripheral fingers, and is therefore the finger with the highest rigidity.

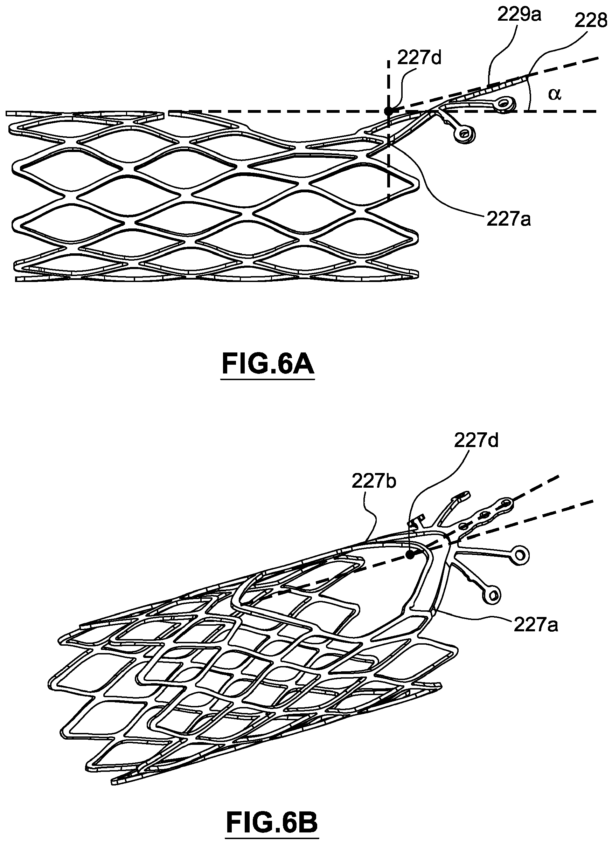

FIG. 6A and 6B show a side view and a top perspective view, respectively, of the uncovered distal tubular part of another example of vascular prosthesis according to the first aspect of the present disclosure, with its flap in the deployed position.

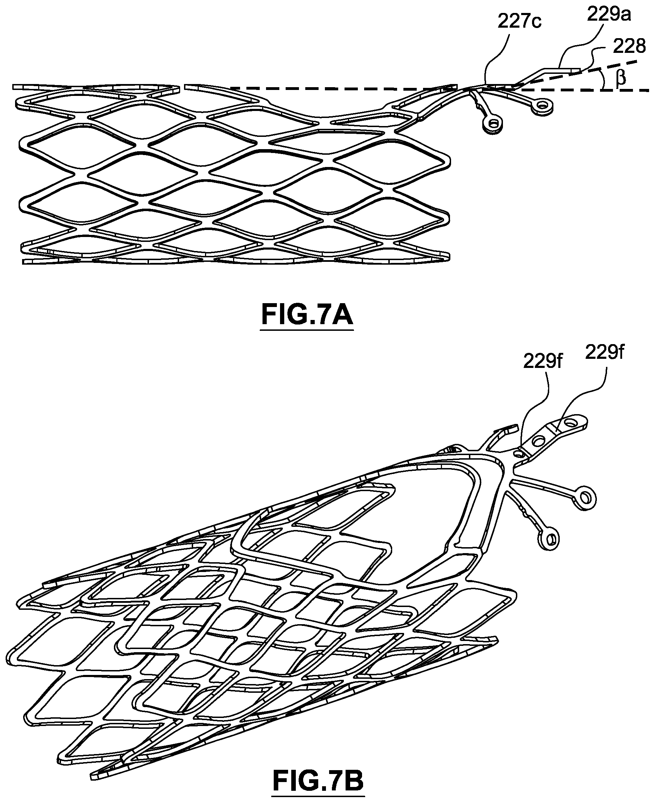

FIG. 7A and 7B show a side view and a top perspective view, respectively, of the uncovered distal tubular part of yet another example of vascular prosthesis according to the first aspect of the present disclosure, with its flap in the deployed position.

In these two latter examples of prostheses, as shown in FIGs 6A and 7A, in the deployed position, the structural frame of the flap 224 (and therefore the flap 222) are outwardly offset with respect to the axial direction of the distal tubular part 20. The offset may for example be between 0.5° and 30° with respect to said axial direction, for example between 5° and 20°. Such offset may for example allow to compensate for the plastification, if present, of certain parts of the prosthesis, by forcing the flap 222 to adhere to the vessel wall.

In the prosthesis illustrated in FIGs. 6A and 6B, the offset is illustrated by the angle α. As shown in the Figures, the angle α between, on the one hand, a straight line passing through the tip 228 of the flap 222 and the circumferential middle point 227d between the ends 227a, 227b of the arched segment 227, and, on the other hand, the axial direction of the distal tubular part, is superior to 0° and is about 20°.

In the case of the prosthesis illustrated in FIGs. 7A and 7B, the middle finger 229a comprises two folds 229f, so that, in the deployed position, the finger 229a extends outwardly from the central axis of the distal tubular part 20. An angle β superior to 0°, illustrated in FIG. 7A, is formed between, on the one hand, a straight line passing through the distal end of the finger 228 and its connection point to the arched segment (i.e., substantially the middle of the arched segment 227c), and, on the other hand, the axial direction of the distal tubular part.

The vascular prosthesis may be implanted by means of an implantation device - not illustrated in the Figures. The implantation device comprises the foregoing vascular prosthesis with the flap being in a folded position, in which the flap is folded inside the second lumen 32. The device further comprises a sheath and a link between the sheath and the vascular prosthesis. The link may be, for instance, a rod, a rope or a combination thereof. For example, the link may pass through all three holes 229x of the middle finger 229a shown in FIGs. 5, 6B and 7B. The sheath incorporating the vascular prosthesis may have a perforating head for penetrating a vessel and carrying out a transparietal puncture in said vessel. The perforating head may include a trocar, a stylet, a needle, a perforating nose, a catheter, a sheath, a probe, a lasso, and combinations thereof. This allows, among other features, the part of the sheath incorporating the vascular prosthesis to be inserted in the vessel.

Once inserted in the transparietal puncture of a vessel and correctly positioned, the flap of the distal tubular part of the vascular prosthesis may be moved to an unfolded position in which the flap is axially deployed, and, either simultaneously or consecutively, the first end portion and intermediate portion may radially expand and adhere to the vessel's wall, so that the vascular prosthesis remains implanted in the vessel, due to the radial force of the first end portion and intermediate portion.

The implantation device may include additional features that may ease the implantation of the vascular prosthesis in the vessel, such as a guide, for example, a hydrophilic guide. During an implantation procedure, such a guide may be used to insert the prosthesis within a vessel, and perform the positioning of the first end portion of the prosthesis in the upstream part of the vessel.

The sheath may further include features that may ease the automatic deployment of the prosthesis, *i.e.,* the axial deployment of the flap and/or the radial expansion of the first end and intermediate portions, such as a tear-away sheath, a ripcord, or mechanical clamps.

The proposed vascular prosthesis is suitable for a wide variety of therapeutic uses. Such therapeutic uses include the anastomosis of vessels, such as blood vessels. The vascular prosthesis can for example be implanted in vessels pertaining to the urethra, trachea, branchi, esophagus, biliary tract, and the like.

The vascular prosthesis aims at improving conventional side-to-end anastomosis techniques, by providing, among other advantages, a less invasive surgical approach. For example, the vascular prosthesis may be used as a bypass graft in percutaneous bypass methods involving the step of implanting the prosthesis between a first vessel and a second vessel in order to fluidly connect said first and second vessels.

The term "percutaneous" refers herein to a step (or series of steps) of a surgical procedure that is (are) performed through the skin, wherein access to vessels, inner organs or other tissue is done via puncture of the skin, as opposed to a procedure performed through conventional open surgery where vessels, inner organs or other tissue are exposed, typically, with the use of a scalpel. The vascular prosthesis may for example be used to perform femoro-popliteal bypasses, which are associated with a complication rate of 37 %, mostly related to the inguinal incision, with an average hospital stay of 10.2 days. The technical benefits of the vascular prosthesis may allow a significant reduction in the rate of complications and a hospital stay divided by two.

In addition, in certain cases, the vascular prosthesis may avoid the use of an inflatable balloon. Moreover, compared to classical open surgery involving the use of clamps, the foregoing examples of vascular prostheses may offer the following non exhaustive list of advantages:
- avoiding inguinal incision (versus 10 cm for surgery);
- avoiding arterial dissection zone (versus 5 cm for surgery);
- avoiding the use of arterial clamps (upstream and downstream the vessel);
- avoiding suture by a vascular thread;
- avoiding the placement of a drain;
- reducing the duration of anastomosis: less than 2 minutes (versus 15-20 minutes).

The examples or embodiments described in this disclosure are illustrative and non-limiting, and a person skilled in the art may easily, in view of this disclosure, modify these examples or embodiments, or consider others, while remaining within the scope of the invention.

In particular, a person skilled in the art will be able to devise variations comprising only some of the features of the previously described examples or embodiments, if these features alone are sufficient to provide one of the advantages of the invention. Furthermore, the various features of these examples or embodiments may be used alone or combined with each other. When combined, these features may be combined as described above or differently, as the invention is not limited to the specific combinations described herein. In particular, unless otherwise specified, a feature described in connection with one example or embodiment may be similarly applied to another example or embodiment.

## Claims

1. A vascular prosthesis (1) configured to be implanted in a vessel, having substantially a T-shape, comprising a proximal tubular part (10) forming the base of the "T" and a distal tubular part (20) forming the head of the "T", wherein:
- the proximal tubular part (10) has a first lumen (31), the distal tubular part (20) has a second lumen (32) and the first and second lumens (31, 32) are fluidly connected to form a common lumen;
- the distal tubular part (20) comprises a first end portion (210), a second end portion (220), and an intermediate portion (230) extending axially between the first and second end portions (210, 220), the intermediate portion being provided with a lateral window (235) from which the proximal tubular part (10) extends, the lateral window being surrounded by a peripheral frame (234b);
- the first end portion (210) and the intermediate portion (230) are radially expandable;
- the second end portion (220) is formed by a flap (222) movable between a folded position in which the flap (222) is folded inside the second lumen (32) and an unfolded position in which the flap (222) is axially deployed, the flap (222) being opposed to a circumferential indentation (223) of the second end portion (220) in the unfolded position;
- the flap (222) has a structural frame (224) comprising an arched segment (227); and
- the arched segment (227) is connected at its ends to the intermediate portion (230), on each side of the peripheral frame (234b), and surrounds a portion of the peripheral frame (234b) in the unfolded position, the arched segment (227) being bendable so as to form a joint for the folding/unfolding movement of the flap (222).

2. The vascular prosthesis of claim 1, wherein the arched segment (227) is elastically bendable between an unstable bent position corresponding to the folded position of the flap (222) and a stable unbent position corresponding to the unfolded position of the flap (222).

3. The vascular prosthesis of claim 1 or claim 2, wherein, in the deployed position, the circumferential width of the flap (222) decreases with the distance from the intermediate portion (230).

4. The vascular prosthesis of any one of the preceding claims, wherein the circumferential distance along the flap (222) between the ends (227a, 227b) of the arched segment is comprised between 10% and 50% of the total circumference of the second lumen (32).

5. The vascular prosthesis of any one of the preceding claims, wherein in the unfolded position, the flap (222) is outwardly offset with respect to the axial direction of the distal tubular part (20).

6. The vascular prosthesis of any one of the preceding claims, wherein the structural frame (224) of the flap (222) further comprises at least one finger (229) connected to the arched segment (227).

7. The vascular prosthesis of claim 6, wherein the at least one finger (229a) extends from the middle of the arched segment (227c).

8. The vascular prosthesis of claim 6 or claim 7, wherein the at least one finger (229) extends perpendicularly from the arched segment (227).

9. The vascular prosthesis of any one of claims 6-8, wherein the at least one finger (229) has a distal end with a hole (229x).

10. The vascular prosthesis of any one of claims 6-9, wherein the at least one finger (229) has a distal end with a widening (229y).

11. Vascular prosthesis according to any one claims 6-10, wherein the at least one finger (229a) comprises at least one fold, so that, in the deployed position, the finger extends outwardly from the central axis of the distal tubular part (20).

12. Implantation device comprising a vascular prosthesis (1) according to any one of claims 1 to 11 with the flap (222) in the folded position, a sheath and a link between the sheath and the vascular prosthesis.
